# EUROPÄISCHE PATENTSCHRIFT

(11) **EP 0 775 684 B1**
(45) Veröffentlichungstag und Bekanntmachung des Hinweises auf die Patenterteilung: **18.08.1999**
(21) Anmeldenummer: 96118157.5
(22) Anmeldetag: 13.11.1996
(51) Int. Cl.: C07C 45/30, C07C 205/44, C07D 307/60, C07D 319/02

(54) **Verfahren zur Oxidation primärer oder sekundärer Alkohole**
Process for the oxidation of primary or secondary alcohols
Procédé pour l'oxydation d'alcools primaires ou secondaires

(30) Priorität: 21.11.1995 CH 329195
(43) Veröffentlichungstag der Anmeldung: 28.05.1997
(73) Patentinhaber: F. HOFFMANN-LA ROCHE AG, 4070 Basel (CH)
(72) Erfinder: Jenny, Christian-Johannes, 4053 Basel (CH); Lohri, Bruno, 4303 Kaiseraugst (CH); Schlageter, Markus, 4103 Bottmingen (CH)
(74) Vertreter: Witte, Hubert, Dr.

(56) Entgegenhaltungen:
- EP-A- 0 574 666
- DE-A- 4 007 923
- JOURNAL OF ORGANIC CHEMISTRY, Bd. 52, Nr. 12, 12.Juni 1987, Seiten 2559-2562, XP000567759 ANELLI P L ET AL: "FAST AND SELECTIVE OXIDATION OF PRIMARY ALCOHOLS TO ALDEHYDES OR TO CARBOXYLIC ACIDS AND OF SECONDARY ALCOHOLS TO KETONES MEDIATED BY OXOAMMONIUM SALTS UNDER TWO-PHASE CONDITIONS"
- J. ORG. CHEM. (JOCEAH);75; VOL.40 (12); PP.1860-2, WALTER REED ARMY INST. RES.;DIV. BIOCHEM.; WASHINGTON; D. C., XP002023694 CELLA J A ET AL: "Nitroxide-catalyzed oxidation of alcohols using m-chloroperbenzoic acid. New method"
- CHEMICAL ABSTRACTS, vol. 116, no. 9, 2.März 1992 Columbus, Ohio, US; abstract no. 083223, TORII S ET AL: "Manufacture of aldehydes from primary alcohols" XP002023695 & JP 03 184 934 A (OSAKA YUKI KAGAKU KOGYO CO., LTD.;JAPAN) 12.August 1991

## Beschreibung

Die vorliegende Erfindung betrifft ein neues Verfahren zur Oxidation primärer oder sekundärer Alkohole.

Alkohole können beispielsweise mit Natriumhypochlorit als Oxidationsmittel und 4-Methoxy-2,2,6,6,-tetramethylpiperidin-1 oxid als Katalysator zu Aldehyden und Ketonen oxidiert werden. (J. Org. Chem. Vol. 52, Nr. 12, 1987, Seite 2559-2562). Dieses Verfahren eignet sich nicht zur Herstellung von Aldehyden und Ketonen, die im Kontakt mit wässrigbasischem Milieu instabil sind.

Der vorliegenden Erfindung liegt nun die Aufgabe zugrunde, ein Verfahren zur Herstellung von Aldehyden und Ketonen bereitzustellen, welches wässrig-basische Bedingungen vermeidet.

Das erfindungsgemässe Verfahren ist dadurch gekennzeichnet, dass man primäre oder sekundäre Alkohole in Gegenwart einer organischen N-Chlorverbindung und einer Verbindung der allgemeinen Formel I oxidiert, 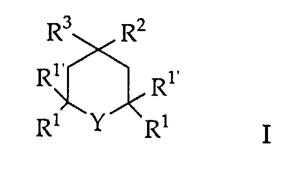 worin
R¹ und R^{1'} gleiche oder verschiedene niedere Alkylgruppen;
R² und R³ entweder beide Reste Wasserstoff oder niederes Alkoxy oder der eine Rest Wasserstoff und der andere Hydroxy, niederes Alkoxy, niederes Alkylcarbonyloxy, niederes Alkylcarbonylamino oder Arylcarbonyloxy oder R² und R³ gemeinsam Ketal-Gruppierungen der Formel a-c 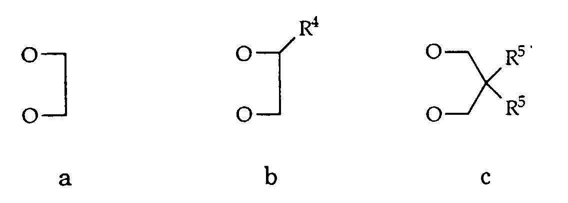
R⁴ niederes Alkyl;
R⁵ und R^{5'} Wasserstoff oder gleiche oder verschiedene niedere Alkylgruppen;
Y eine Gruppierung der allgemeinen Formel d-f 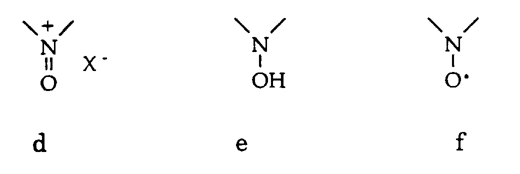
und X⁻ ein Anion bedeuten.

Als Oxidationsmittel geeignete organische N-Chlorverbindungen können im Rahmen der vorliegenden Erfindung z.B. N-Chlor-4-toluolsulfonamid-Na-salz(Chloramin T), N-Chlor-benzolsulfonamid-Na-salz (Chloramin B), Trichlorisocyanursäure oder Dichlordimethylhydantoin sein. Bevorzugt ist Trichlorisocyanursäure oder Dichlordimethylhydantoin.

Der Ausdruck "niederes Alkyl" umfasst geradkettige oder verzweigte gesättigte Kohlenwasserstoffreste mit bis zu 7 Kohlenstoffatomen, wie Methyl, Ethyl, n-Propyl, Isopropyl, n-Butyl, sek.Butyl, Isobutyl, n-Pentyl, n-Hexyl und dergleichen.

Der Ausdruck "niederes Alkoxy" bezeichnet über ein Sauerstoffatom gebundene niedere Alkylgruppen im Sinne der vorstehenden Definition, wie z.B. Methoxy, Butoxy, Hexoxy.

Der Ausdruck "niederes Alkylcarbonyloxy" bezeichnet über ein Sauerstoffatom gebundene niedere Alkylcarbonylreste. Der Ausdruck "nieder Alkylcarbonyl" bezeichnet über eine Carbonylgruppe gebundene niedere Alkylgruppen und umfasst im Sinne der vorstehenden Definition Reste wie Acetyl, Propionyl und dergleichen.

Der Ausdruck "niederes Alkylcarbonylamino" bezeichnet über ein Stickstoffatom gebundene niedere Alkylcarbonylreste, wie z. B. Acetylamino.

Der Ausdruck "Arylcarbonyloxy" bezeichnet über ein Sauerstoffatom gebundene Arylcarbonylreste. Der Ausdruck "Arylcarbonyl" bezeichnet über eine Carbonylgruppe gebundene Arylgruppen. Der Ausdruck "Aryl" bezeichnet gegebenenfalls substituierte aromatische Reste, wie beispielsweise einen gegebenenfalls substituierten Phenylrest, wobei als Substituenten z.B. niedere Alkylgruppen oder niedere Alkoxygruppen in Betracht kommen können.

Das Verfahren eignet sich zur Oxidation von primären oder sekundären Alkylalkoholen mit geradkettiger oder verzweigter Kohlenwasserstoffkette wie z.B. 1-Octanol, 2-Octanol, 1-Decanol; Cycloalkylalkoholen wie z.B. Cyclohexanol; aliphatischen Alkoholen, die einen aromatischen Substituenten tragen wie z.B. Phenylethanol, Benzylalkohol und substituierten Benzylalkoholen.

Das Verfahren eignet sich auch zur Oxidation von heterocyclischen Alkoholen, insbesondere zur Oxidation von Hydroxylactonen wie 2-Hydroxy-3,3-dimethyl-γ-butyrolacton (Pantolacton) sowie von empfindlichen Alkoholen mit einer Peroxidfunktion wie z.B. von (1S,4R,5R,8S)-und (1S,4S,5R,8S)-4-(Hydroxymethyl)-4,8-dimethyl-2,3-dioxabicyclo[3.3.1] nonan-7-on.

Erfindungsgemäss können der Alkohol und die jeweilige N-Chlor-Verbindung in einem organischen Lösungsmittel suspendiert werden und die Oxidation durch Zugabe einer Lösung des Katalysators der Formel I initiiert werden. Es können aber auch der Alkohol und der Katalysator vorgelegt werden und das Oxidationsmittel zugetropft werden. Die Reihenfolge der Reaktanden ist nicht kritisch.

Die Umsetzung erfolgt in einem Molverhältnis von Alkohol zu Oxidationsmittel von etwa 1:1 bis 1:1.25 (bezogen auf Aktiv-Chlor).

Der Katalysatorzusatz ist für das erfindungsgemässe Oxidationsverfahren essentiell. Bevorzugt sind Katalysatoren der Formel I, worin R¹ und R^{1'} Methyl; R² und R³ entweder beide Reste Wasserstoff, oder der eine Rest Wasserstoff und der andere Acetylamino oder R² und R³ gemeinsam Ketal-Gruppierungen der Formel a-c; R⁴ Ethyl; R⁵ und R⁵' Methyl; Y eine Gruppierung der allgemeinen Formel f und X⁻ ein Anion bedeuten.

Die Verbindungen der allgemeinen Formel I sind bekannte Verbindungen, deren Herstellung beispielsweise in den Europäischen Patentanmeldungen EPA-0 574 666 und EPA-0 574 667 oder in Synthesis, 1971, S.190 ff beschrieben sind. Aus EP-A-0 574 666 ist ferner bekannt, dass diese Verbindungen in stark basischem Medium stabil sind und als Redox-Katalysatoren in zweiphasigen, wässrig-organischen Systemen verwendet werden können.

Die Oxidation in Gegenwart von Verbindungen der Formel I erfolgt mit einer Menge von Verbindungen der Formel I von 0.05 -20 Mol%, bevorzugt 0.1-1 Mol% bezüglich eingesetztem Alkohol.

Geeignete Lösungsmittel können im Rahmen der vorliegenden Erfindung Methylenchlorid, Essigester, Aceton, Chloroform, Butylacetat, Diethylether, tert. Butylmethylether, Dichlorethan und dergleichen sein. Bevorzugt sind Methylenchlorid, Aceton und Essigester.

Das Oxidationssystem enthält eine Base zur Neutralisation der entstehenden Salzsäure z.B. Natriumacetat, Natriumbicarbonat oder Pyridin.

Die Oxidation kann zweckmässigerweise in einem Temperaturbereich von (-15) °C - 50 °C , vorzugsweise bei 0 °C - 5 °C erfolgen. Zweckmässigerweise arbeitet man unter Atmosphärendruck.

Die folgenden Beispiele erläutern die Erfindung näher, sollen aber keinerlei Einschränkung darstellen.

Nachfolgend sind die in den Beispielen verwendeten Abkürzungen der eingesetzten Tetramethylverbindungen der Formel I aufgeführt.

| **Katalysator** | **Name** |
|---|---|
| TEMPO (Verbindung A) | 2,2,6,6-Tetramethyl-1-piperidinyloxyl-Radikal |
| | |
| TEMPO-Derivat B | 7,7,9,9,-Tetramethyl-1,4-dioxa-8-azaspiro[4,5]dec-8-yloxyl-Radikal |
| | |
| TEMPO-Derivat C | (R,S)-2-Ethyl-7,7,9,9-tetramethyl-1,4-dioxa-8-azaspiro [4,5]dec-8-yloxyl-Radikal |
| | |
| TEMPO-Derivat D | 3,3,8,8,10,10-Hexamethyl-1,5-dioxa-9-azaspiro[5,5] undec-9-yloxyl-Radikal |
| | |
| TEMPO-Derivat E | 4-(Acetylamino)-2,2,6,6-tetramethyl-1-piperidinyloxyl-Radikal |

### I Oxidation von Alkylalkoholen

### Beispiel 1:

### Herstellung von Octanal

3,5 g (15,1 mmol) Trichlorisocyanursäure, 3,7 g (45,1 mmol) Natriumacetat und 10 mg TEMPO (0,06 mmol) wurden in einem 100-ml-Sulfierkolben in 40 ml Methylenchlorid suspendiert und die Suspension unter Rühren auf (-7) - (-9) ^{o}C abgekühlt. Eine Lösung von 5 g 1-Octanol (38,4 mmol) in 20 ml Methylenchlorid wurde innerhalb von 20 Minuten zudosiert, worauf das Gemisch 80 Minuten bei (-7) - (-9) ^{o}C gehalten wurde. Danach war die Reaktion beendet. Zur Aufarbeitung wurde der weisse Niederschlag abfiltriert und das Filtrat mit Natriumbicarbonatlösung sowie mit Kochsalzlösung gewaschen. Die Destillation des Rohproduktes ergab 4,5 g (91%) Octanal, GC-Gehalt: (98,2%) (Flächenprozente).

### Beispiel 2:

### Herstellung von 2-Octanon

5 g 2-Octanol (38,4 mmol) wurde wie in Beispiel 1 beschrieben oxidiert. Die Destillation des Rohproduktes ergab 4,77 g (96%) 2-Octanon, GC-Gehalt: (99,5%) (Flächenprozente).

### Beispiel 3:

### Herstellung von Decanal

6.08 g (38.4 mmol) 1-Decanol wurden in einem 200-ml-Sulfierkolben in 58 ml Methylenchlorid gelöst. 3.3 g (40.2 mmol) Natriumacetat sowie 3.6 g (15.5 mmol) Trichlorisocyanursäure wurden hinzugefügt. Das Gemisch wurde unter Rühren auf 0 ^{o}C abgekühlt. Eine Lösung von 30 mg (0.12 mmol) TEMPO-Derivat C in 3.5 ml Methylenchlorid wurde innerhalb von 30 Minuten zudosiert. Durch konstantes Kühlen wurde die Temperatur bei 0-3 ^{o}C gehalten. Nach 1 Stunde war die Reaktion beendet. Der weisse Niederschlag wurde abfiltriert. Das Filtrat wurde wässrig aufgearbeitet. Als Rohprodukt wurden 5.9 g (98%) Decanal als farblose Flüssigkeit erhalten. GC-Gehalt: 97% (Flächenprozente).

### Beispiel 4:

### Herstellung von Cyclohexanon

5 g Cyclohexanol (49,9 mmol), 4,1 g (50 mmol) Natriumacetat und 10 mg TEMPO (0,06 mmol) wurden in einem 100-ml-Sulfierkolben in 40 ml Methylenchlorid suspendiert und die Suspension unter Rühren auf (-1) - 2°C abgekühlt. Eine Lösung von 4,6 g (19,8 mmol) Trichlorisocyanursäure in 20 ml Aceton wurde innerhalb von 20 Minuten zudosiert, worauf das Gemisch 2,5 Stunden bei (-1) - 2 ^{o}C gehalten wurde. Danach war die Reaktion beendet, ohne nennenswerte Chlorierung in 2-Stellung. Der weisse Niederschlag wurde abfiltriert und das Filtrat wurde mit Natriumbicarbonatlösung sowie mit Kochsalzlösung gewaschen. Die Destillation des Rohproduktes ergab 3,97 g (81%) Cyclohexanon, GC-Gehalt: 98,2% Cyclohexanon, 0,8% 2-Chlorcyclohexanon (Flächenprozente).

### II Oxidation von aromatischen Alkoholen

### Beispiel 5:

### Herstellung von Benzaldehyd

### (Trichlorisocyanursäure und TEMPO)

4,3 g (18,5 mmol) Trichlorisocyanursäure, 3,8 g Natriumacetat (46,2 mmol) und 10 mg TEMPO (0,06 mmol) wurden in einem 100-ml-Sulfierkolben in 40 ml Methylenchlorid suspendiert und die Suspension unter Rühren auf (-7) -(-9) ^{o}C abgekühlt. Eine Lösung von 5 g Benzylalkohol (46,2 mmol) in 20 ml Methylenchlorid wurde innerhalb von 20 Minuten zudosiert, worauf das Gemisch 20 Minuten bei (-7) -(-9) ^{o}C gehalten wurde. Danach war die Reaktion beendet. Der weisse Niederschlag wurde abfiltriert und das Filtrat wurde mit Natriumbicarbonatlösung sowie mit Kochsalzlösung gewaschen. Die Destillation des Rohproduktes ergab 4,35 g (88,6%) Benzaldehyd, GC-Gehalt: (97,7%) (Flächenprozente).

### Beispiel 6:

### Herstellung von Benzaldehyd

### (Trichlorisocyanursäure und TEMPO Derivat C)

4.15 g (38.4 mmol) Benzylalkohol wurden in einem 200-ml-Sulfierkolben in 58 ml Methylenchlorid gelöst. 3.3 g (40.2 mmol) Natriumacetat sowie 3.6 g (15.5 mmol) Trichlorisocyanursäure wurden hinzugefügt. Das Gemisch wurde unter Rühren auf 0 ^{o}C abgekühlt. Eine Lösung von 30 mg (0.12 mmol) TEMPO-Derivat C in 3.5 ml Methylenchlorid wurde innerhalb von 30 Minuten zudosiert. Durch konstantes Kühlen wurde die Temperatur bei 0-3 ^{o}C gehalten. Nach 1 Stunde war die Reaktion beendet. Der weisse Niederschlag wurde abfiltriert. Das Filtrat wurde wässrig aufgearbeitet. Als Rohprodukt wurden 4.0 g (98%) Benzaldehyd erhalten, GC-Gehalt: 99.2%. Die Destillation des Rohprodukts ergab 3.22 g (79%) Benzaldehyd, GC-Gehalt: 99.3% (Flächenprozente).

### Beispiel 7:

### Herstellung von Benzaldehyd

### (1,3-Dichlor-5,5-dimethylhydantoin und TEMPO)

5,4 g (27,4 mmol) 1,3-Dichlor-5,5-dimethylhydantoin, 3,8 g Natriumacetat (46,2 mmol) und 5 g Benzylalkohol (46,2 mmol) wurden in einem 100-ml-Sulfierkolben in 40 ml Methylenchlorid suspendiert, die Suspension unter Rühren auf 0 ^{o}C abgekühlt und mit 10 mg TEMPO (0,06 mmol) versetzt, worauf das Gemisch 80 Minuten bei 0 ^{o}C gehalten wurde. Danach liess man die Temperatur innerhalb von 1 Stunde auf 25 °C ansteigen. Bei 25 °C wurde das Gemisch 16 Stunden weitergerührt. Danach war die Reaktion beendet. Der weisse Niederschlag wurde abfiltriert und das Filtrat wurde mit Natriumbicarbonatlösung sowie mit Kochsalzlösung gewaschen. Die Destillation des Rohproduktes ergab 4,0 g (81,5%) Benzaldehyd, GC-Gehalt: (99,7%) (Flächenprozente).

### Beispiel 8:

### Herstellung von 4-Nitrobenzaldehyd

5 g 4-Nitrobenzylalkohol (32,6 mmol), 2,7 g (32,9 mmol) Natriumacetat und 10 mg TEMPO (0,06 mmol) wurden in einem 100-ml-Sulfierkolben in 40 ml Methylenchlorid suspendiert und die Suspension unter Rühren auf (-8)-(-10) ^{o}C abgekühlt. Eine Lösung von 3,03 g (13,0 mmol) Trichlorisocyanursäure in 20 ml Aceton wurde innerhalb von 1 Stunde zudosiert, worauf das Gemisch 1 Stunde bei (-5) ^{o}C gehalten wurde. Danach war die Reaktion beendet. Der weisse Niederschlag wurde abfiltriert und das Filtrat wurde mit Natriumbicarbonatlösung sowie mit Kochsalzlösung gewaschen. Die organische Phase wurde eingeengt und der Rückstand aus Diisopropylether umkristallisiert. Es resultierten 4,62 g (94%) 4-Nitrobenzaldehyd, GC Gehalt: (97,7%) (Flächenprozente).

### Beispiel 9:

### Herstellung von Acetophenon

4.7 g (38.4 mmol) rac.-1-Phenylethanol und 3.18 g (40.2 mmol) Pyridin wurden unter Inertgas in einem 200-ml-Sulfierkolben in 60 ml Essigester gelöst. Das Gemisch wurde unter Rühren auf 0 ^{o}C abgekühlt und mit 60 mg (0.28 mmol) TEMPO-Derivat E versetzt. Danach wurde eine Lösung von 3.6 g (15.5 mmol) Trichlorisocyanursäure in 38 ml Essigester innerhalb von 1 Stunde bei einer Temperatur von 0-3 ^{o}C zudosiert. Das Reaktionsgemisch wurde noch 1 Stunde bei 0-3 ^{o}C weitergerührt. Danach war die Reaktion beendet. Der weisse Niederschlag wurde abfiltriert, und das Filtrat wurde zur Reduktion der überschüssigen Trichlorisocyanursäure mit einer Lösung von Natriumpyrosulfit behandelt. Das Filtrat wurde mit Natriumbicarbonatlösung sowie mit Kochsalzlösung gewaschen. Als Rohprodukt wurden 4.7 g (102%) Acetophenon erhalten, GC-Gehalt: 99.8%. Die Destillation des Rohprodukts ergab 3.19 g (69%) Acetophenon, GC-Gehalt: 99.9% (Flächenprozente).

### Beispiel 10:

### Herstellung von Anisaldehyd

### (Trichlorisocyanursäure)

5 g 4-Anisalkohol (36,2 mmol), 3,0 g (36,6 mmol) Natriumacetat und 10 mg TEMPO (0,06 mmol) wurden in einem 100-ml-Sulfierkolben in 40 ml Methylenchlorid suspendiert und die Suspension unter Rühren auf (-8)-(-10) ^{o}C abgekühlt. Eine Lösung von 3,4 g (14,5 mmol) Trichlorisocyanursäure in 20 ml Aceton wurde innerhalb von 40 Minuten zudosiert, worauf das Gemisch 1 Stunde bei 0 ^{o}C gehalten wurde. Danach war die Reaktion beendet. Der weisse Niederschlag wurde abfiltriert und das Filtrat wurde mit Natriumbicarbonatlösung sowie mit Kochsalzlösung gewaschen. Die organische Phase wurde eingeengt. Es resultierten 5,2 g Rohprodukt als Gemisch aus Anisaldehyd und 4-Chloranisol. GC-Gehalt: 46,7% 4-Chloranisol, 20,7% Anisaldehyd (Flächenprozente).

### Beispiel 11:

### Herstellung von Anisaldehyd

### (1,3-Dichlor-5,5-dimethylhydantoin)

5 g Anisalkohol (36,2 mmol), 3,0 g (36,6 mmol) Natriumacetat und 4,2 g (21,3 mmol) Dichlordimethylhydantoin wurden in einem 100-ml-Sulfierkolben in 40 ml Methylenchlorid suspendiert, die Suspension unter Rühren auf 0 ^{o}C abgekühlt und mit 10 mg TEMPO (0,06 mmol) versetzt, worauf das Gemisch 16 Stunden bei 0 ^{o}C gehalten wurde. Danach war die Reaktion beendet, ohne dass 4-Chlor-anisol in nennenswerter Menge entstanden war. Der weisse Niederschlag wurde abfiltriert und das Filtrat wurde mit Natriumbicarbonatlösung sowie mit Kochsalzlösung gewaschen. Die Destillation des Rohproduktes ergab 4,49 g (91%) Anisaldehyd GC-Gehalt: 88,8% (Flächenprozente).

### Beispiel 12:

### Herstellung von Dihydro-4,4-dimethyl-2,3-furandion (Ketopantolacton)

### (TEMPO)

5 g (38.4 mmol) 2-Hydroxy-3,3-dimethyl-γ-butyrolacton (Pantolacton) wurden in einem 200-ml-Sulfierkolben in 118 ml Methylenchlorid gelöst. 3.3 g (40.2 mmol) Natriumacetat sowie 3.6 g (15.5 mmol) Trichlorisocyanursäure wurden hinzugefügt. Das Gemisch wurde unter Rühren auf 0 ^{o}C abgekühlt. Eine Lösung von 38.2 mg (0.24 mmol) TEMPO in 2 ml Methylenchlorid wurde innerhalb von 10 Minuten zudosiert. Durch konstantes Kühlen wurde die Temperatur bei 0-3 °C ge-halten. Nach 7 Stunden Reaktionszeit wurde der weisse Niederschlag abfiltriert. Das Filtrat wurde eingeengt. Chromatographie des Rückstandes (SiO₂, Toluol/Essigester 85:15) und anschliessende Umkristallisation des chromatographierten Produkts ergab 4.23 g (86%) Ketopantolacton, Smp. 67.5-68 ^{o}C, GC-Gehalt: 100% (Flächenprozente).

### Beispiel 13:

### Herstellung von Dihydro-4,4-dimethyl-2,3-furandion (Ketopantolacton)

### (TEMPO-Derivat B)

5 g (38.4 mmol) 2-Hydroxy-3,3-dimethyl-γ-butyrolacton (Pantolacton) wurden in einem 200-ml-Sulfierkolben in 118 ml Methylenchlorid gelöst. 3.3 g (40.2 mmol) Natriumacetat sowie 3.6 g (15.5 mmol) Trichlorisocyanursäure wurden hinzugefügt. Das Gemisch wurde unter Rühren auf 0 ^{o}C abgekühlt. Eine Lösung von 60 mg (0.28 mmol) TEMPO-Derivat B in 2 ml Methylenchlorid wurde innerhalb von 10 Minuten zudosiert. Durch konstantes Kühlen wurde die Temperatur bei 0-3 ^{o}C gehalten. Nach 3 Stunden war die Reaktion beendet. Der weisse Niederschlag wurde abfiltriert und das Filtrat wurde zur Reduktion der überschüssigen Trichlorisocyanursäure mit Natriumbisulfitlösung behandelt. Aufarbeitung des Reaktionsgemisches und anschliessende Umkristallisation des Rohprodukts ergab 3.5 g (71%) Ketopantolacton, GC-Gehalt: 99.6% (Flächenprozente).

### Beispiel 14:

### Herstellung von Dihydro-4,4-dimethyl-2,3-furandion (Ketopantolacton)

### (TEMPO-Derivat C)

5 g (38.4 mmol) Pantolacton wurden wie in Beispiel 13 beschrieben mit 3.6 g (15.5 mmol) Trichlorisocyanursäure umgesetzt. Im Unterschied zu Beispiel 13 wurden als Katalysator 60 mg (0.25 mmol) TEMPO-Derivat C eingesetzt. Die Reaktion war nach 3 Stunden beendet. Erhalten wurden nach Umkristallisation des Rohprodukts 4.6 g (93%) Ketopantolacton, Smp. 69-70 ^{o}C, GC-Gehalt: 100% (Flächenprozente).

### Beispiel 15:

### Herstellung von Dihydro-4,4-dimethyl-2,3-furandion (Ketopantolacton)

### (TEMPO-Derivat D)

5 g (38.4 mmol) Pantolacton wurden wie in Beispiel 13 beschrieben mit 3.6 g (15.5 mmol) Trichlorisocyanursäure umgesetzt. Im Unterschied zu Beispiel 13 wurden als Katalysator 63 mg (0.24 mmol) Tempo-Derivat D eingesetzt. Die Reaktion war nach 1 Stunde beendet. Erhalten wurden nach Umkristallisation des Rohprodukts 4.85 g (98%) Ketopantolacton, Smp. 68-69 ^{o}C, GC-Gehalt: 100% (Flächenprozente).

### Beispiel 16:

### Herstellung von Dihydro-4,4-dimethyl-2,3-furandion (Ketopantolacton)

### (TEMPO-Derivat C, Essigester als Lösungsmittel)

2.5 g (19.2 mmol) Pantolacton wurden analog Beispiel 14 mit 1.8 g (7.7 mmol) Trichlorisocyanursäure in Gegenwart von 30 mg (0.12 mmol) TEMPO-Derivat C und 1.65 g (20.1 mmol) Natriumacetat umgesetzt. Im Unterschied zu Beispiel 14 wurde als Lösungsmittel Essigester (20 ml) verwendet. Die Reaktion war nach 2 Stunden beendet. Erhalten wurden nach Umkristallisation des Rohprodukts 2.2 g (89%) Ketopantolacton, Smp. 68-69 ^{o}C, GC-Gehalt: 100% (Flächenprozente).

### III Oxidation eines Gemisches von Peroxoalkoholen

### Beispiel 17:

### Herstellung von (1S,4R,5R,8S)-4,8-Dimethyl-7-oxo-2,3-dioxabicyclo[3.3.1] nonan-4-carboxaldehyd und (1S,4S,5R,8S)-4,8-Dimethyl-7-oxo-2,3-dioxabicyclo[3.3.1] nonan-4-carboxaldehyd

183,8 g Alkoholgemisch (HPLC Gehalt 46,2% (1S,4R,5R,8S)-4-(Hydroxymethyl)-4,8-dimethyl-2,3-dioxabicyclo[3.3.1] nonan-7-on + 47,5% (1S,4S,5R,8S)-4-(Hydroxymethyl)-4,8-dimethyl-2,3-dioxabicyclo[3.3.1] nonan-7-on, max. 919 mmol), 77,7 g (940 mmol) Natriumacetat und 84,7 g Trichlorisocyanursäure (346 mmol) wurden in einem 4500-ml-Sulfierkolben in 2700 ml Methylenchlorid suspendiert, die Suspension unter Rühren auf 0⁰ abgekühlt und dort innerhalb von 5 Minuten mit einer Lösung aus 368 mg TEMPO (2,35 mmol) in 10 ml Methylenchlorid versetzt. Das Gemisch wurde 5 Stunden bei 0-3 ^{o}C gehalten. Danach war die Reaktion beendet. Der weisse Niederschlag wurde abfiltriert und das Filtrat wurde mit Natriumsulfatlösung gewaschen. Rohprodukt 169,3 g (>90%)

1:1 Gemisch der Aldehyde als feste gelbliche Masse. (HPLC- Gehalt: 42,3% (1S,4R,5R,8S)-4,8-Dimethyl-7-oxo-2,3-dioxabicyclo[3.3.1] nonan-4-carboxaldehyd, 45,8% (1S,4S,5R,8S)-4,8-Dimethyl-7-oxo-2,3-dioxabicyclo[3.3.1] nonan-4-carboxaldehyd)

### Beispiel 18: (Vergleichsbeispiel)

### Herstellung von Dihydro-4,4-dimethyl-2,3-furandion (Ketopantolacton) ohne TEMPO-Katalysator.

1.18 g (9.1 mmol) Pantolacton wurden in einem 50-ml-Reaktionskolben bei 22 ^{o}C in 27 ml Methylenchlorid gelöst. 777 mg (9.5 mmol) Natriumacetat sowie 850 mg (3.7 mmol) Trichlorisocyanursäure wurden hinzugefügt. Das Gemisch wurde unter Rühren auf 0 ^{o}C abgekühlt. Nach 6 Stunden Reaktionszeit bei 0 ^{o}C wurden 18.5% Ketopantolacton erhalten. Nach einer weiteren Reaktionszeit von 20 Stunden bei 22 ^{o}C betrug die Zusammensetzung 22.2% Ketopantolacton und 77.5% Pantolacton, (Flächenprozente).

## Patentansprüche

1. Verfahren zur Oxidation primärer und sekundärer Alkohole dadurch gekennzeichnet, dass man die Alkohole in Gegenwart einer organischen N-Chlorverbindung und einer Verbindung der allgemeinen Formel I oxidiert, 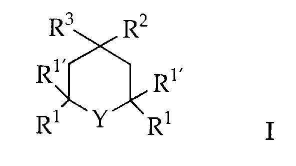 worin
R¹ und R^{1'} gleiche oder verschiedene C₁-C₇-Alkylgruppen;
R² und R³ entweder beide Reste Wasserstoff oder C₁-C₇ Alkoxy; oder der eine Rest Wasserstoff und der andere Hydroxy, C₁-C₇ Alkoxy, C₁-C₇ Alkylcarbonyloxy, Arylcarbonyloxy oder C₁-C₇ Alkylcarbonylamino; oder R² und R³ gemeinsam Ketal-Gruppierungen der Formel a-c 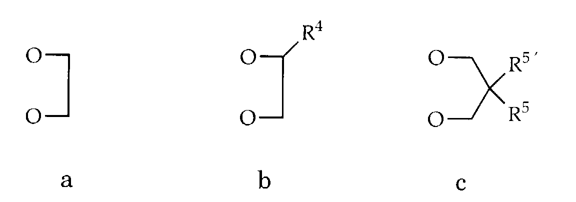
R⁴ C₁-C₇-Alkylgruppe
R⁵ und R^{5'} Wasserstoff oder gleiche oder verschiedene C₁-C₇ Alkyl-gruppen;
Y eine Gruppierung der allgemeinen Formel d-f 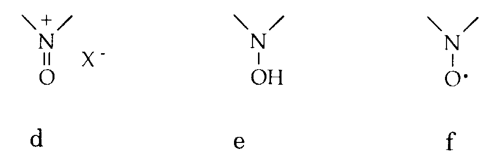
und X⁻ ein Anion bedeuten, wobei C₁-C₇ Alkyl- und C₁-C₇ Alkoxygruppen 1-7 C-Atome umfassen.

2. Verfahren gemäss Anspruch 1, dadurch gekennzeichnet, dass man Verbindung der allgemeinen Formel I verwendet, worin R¹ und R^{1'} Methyl; R² und R³ entweder beide Reste Wasserstoff, oder der eine Rest Wasserstoff und der andere Acetylamino oder R² und R³ gemeinsam Ketal-Gruppierungen der Formel a-c; R⁴ Ethyl; R⁵ und R^{5'} Methyl; Y eine Gruppierung der allgemeinen Formel f und X- ein Anion bedeuten.

3. Verfahren gemäss Anspruch 1 oder 2, dadurch gekennzeichnet, dass die Verbindung der allgemeinen Formel I in einer Menge von 0,05-20 Mol%, insbesondere 0,1-1 Mol% bezogen auf den Alkohol eingesetzt wird.

4. Verfahren gemäss einem der Ansprüche 1-3, dadurch gekennzeichnet, dass als Oxidationsmittel Trichlorisocyanursäure oder Dichlordimethylhydantoin eingesetzt wird.

5. Verfahren gemäss einem der Ansprüche 1-4, dadurch gekennzeichnet, dass das Molverhältnis von Alkohol zu Oxidationsmittel etwa 1:1 bis 1:1.25 (bezogen auf Aktiv-Chlor) beträgt.

6. Verfahren gemäss einem der Ansprüche 1-5, dadurch gekennzeichnet, dass die Reaktion bei einer Temperatur von (-15) °C - 50 °C, vorzugsweise bei 0 °C - 5 °C durchgeführt wird.

7. Verfahren gemäss einem der Ansprüche 1-6, dadurch gekennzeichnet, dass Hydroxylactone oxidiert werden.

8. Verfahren gemäss einem der Ansprüche 1-7, dadurch gekennzeichnet, dass Pantolacton oxidiert wird.

9. Verfahren gemäss einem der Ansprüche 1-6, dadurch gekennzeichnet, dass Alkohole mit einer Peroxidfunktion oxidiert werden.

## Claims

1. A process for the oxidation of primary and secondary alcohols, characterized by oxidizing said alcohols in the presence of an organic N-chloro compound and a compound of general formula I 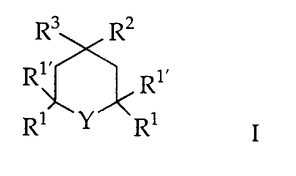 wherein
R¹ and R^{1'} signify the same or different C1-C7 alkyl groups;
R² and R³ either both signify hydrogen or C1-C7 alkoxy or one signifies hydrogen and the other signifies hydroxy, C1-C7 alkoxy, C1-C7 alkylcarbonyloxy, arylcarbonyloxy or C1-C7 alkylcarbonylamino; or R² and R³ together signify ketal groupings of formula a-c 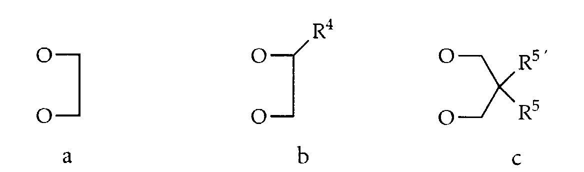
R⁴ signifies C1-C7 alkyl;
R⁵ and R^{5'} signify hydrogen or the same or different C1-C7 alkyl groups;
Y signifies a grouping of general formula d-f 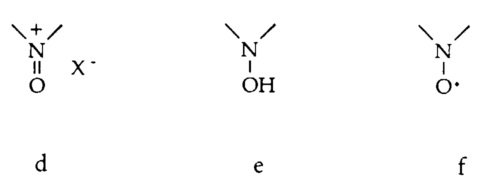 and X⁻ signifies an anion, whereby C1-C7 alkyl and C1-C7 alkoxy groups embrace 1-7 atoms.

2. A process according to claim 1, characterized in that a compound of general formula I is used in which R¹ and R^{1'} signify methyl; R² and R³ either both signify hydrogen or one signifies hydrogen and the other signifies acetylamino or R² and R³ together signify ketal groupings of formula a-c; R⁴ signifies ethyl; R⁵ and R^{5'} signify methyl; Y signifies a grouping of general formula f and X- signifies an anion.

3. A process according to claim 1 or claim 2, characterized in that the compound of general formula I is used in an amount of 0.05-20 mol%, especially 0.1-1 mol%, based on the alcohol.

4. A process according to any one of claims 1-3, characterized in that trichloroisocyanuric acid or dichlorodimethylhydantoin is used as the oxidizing agent.

5. A process according to any one of claims 1-4, characterized in that the molar ratio of alcohol to oxidizing agent is about 1:1 to 1:1.25 (based on active chlorine).

6. A process according to any one of claims 1-5, characterized in that the oxidation is carried out at a temperature of (-15)°C-50°C, preferably at 0°C-5°C.

7. A process according to any one of claims 1-6, characterized in that hydroxylactones are oxidized.

8. A process according to any one of claims 1-7, characterized in that pantolactone is oxidized.

9. A process according to any one of claims 1-6, characterized in that an alcohol having a peroxide function is oxidized.

## Revendications

1. Procédé pour oxyder des alcools primaires et secondaires, caractérisé en ce que l'on oxyde les alcools en présence d'un composé organique N-chloré et d'un composé de formule générale I : 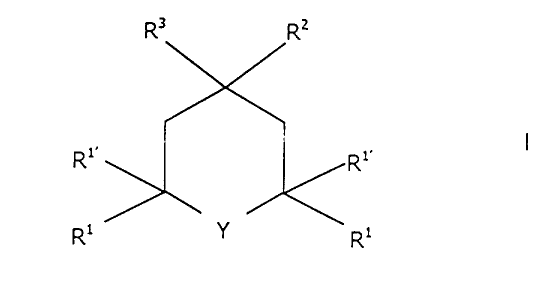 dans laquelle
R¹ et R^{1'} représentent des groupes alkyle en C₁-C₇ identiques ou différents ;
R² et R³ représentent tous deux l'hydrogène ou des groupes alcoxy en C₁-C₇ ; ou bien l'un des deux symboles représentent l'hydrogène et l'autre un groupe hydroxy, alcoxy en C₁-C₇, alkylcarbonyloxy en C₁-C₇, arylcarbonyloxy ou alkylcarbonylamino en C₁-C₇ ; ou bien R² et R³ représentent ensemble un groupement acétal de formule (a) à (c) : 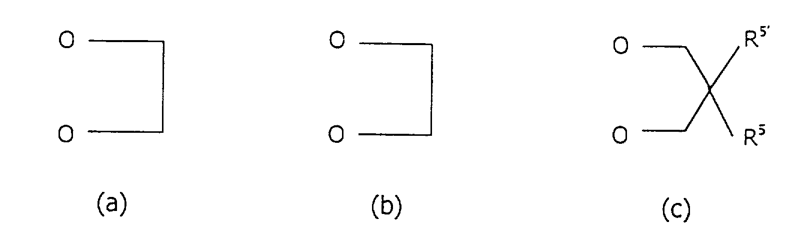
R⁴ représente un groupe alkyle en C₁-C₇ ;
R⁵ et R^{5'} représentent l'hydrogène ou des groupes alkyle en C₁-C₇ identiques ou différents ;
Y représente un groupement général (d) à (f) : 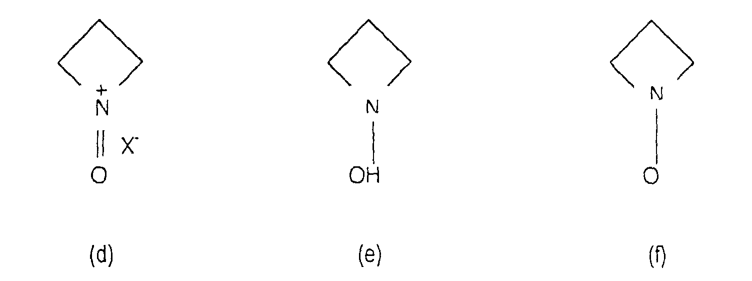
et X⁻ représente un anion, les groupes alkyle et alcoxy en C₁-C₇ contenant de 1 à 7 atomes de carbone.

2. Procédé selon la revendication 1, caractérisé en ce que l'on utilise un composé de formule générale I dans laquelle R¹ et R^{1'} représentent des groupes méthyle ; R² et R³ représentent tous deux l'hydrogène ou bien l'un des deux symboles représente l'hydrogène et l'autre un groupe actylamino ou bien R² et R³ représentent ensemble un groupement acétal de formule (a) à (c), R⁴ représente un groupe éthyle ; R⁵ et R^{5'} représentent des groupes méthyle ; Y représente un groupement de formule générale (f) et X⁻ représente un anion.

3. Procédé selon la revendication 1 ou 2, caractérisé en ce que le composé de formule générale I est mis en oeuvre en quantité de 0,05 à 20 mol %, plus spécifiquement de 0,1 à 1 mol % par rapport à l'alcool.

4. Procédé selon l'une des revendications 1 à 3, caractérisé en ce que l'on utilise en tant qu'agent oxydant l'acide trichlorisocyanurique ou la dichlorodiméthylhydantoïne.

5. Procédé selon l'une des revendications 1 à 4, caractérisé en ce que le rapport molaire alcool/agent oxydant va d'environ 1:1 à 1 :1,25 (rapporté au chlore actif).

6. Procédé selon l'une des revendications 1 à 5, caractérisé en ce que la réaction est effectuée à une température allant de -15 à +15°C, de préférence de 0 à 5°C.

7. Procédé selon l'une des revendications 1 à 6, caractérisé en ce que l'on oxyde des hydroxylactones.

8. Procédé selon l'une des revendications 1 à 7, caractérisé en ce que l'on oxyde la pentolactone.

9. Procédé selon l'une des revendications 1 à 6, caractérisé en ce que l'on oxyde des alcools portant une fonction peroxyde.
